# EUROPÄISCHE PATENTSCHRIFT

(11) **EP 1 032 307 B1**
(45) Veröffentlichungstag und Bekanntmachung des Hinweises auf die Patenterteilung: **02.05.2007**
(21) Anmeldenummer: 98965713.5
(22) Anmeldetag: 30.11.1998
(51) Int. Cl.: A61B 5/15, A61B 5/00

(54) **ANALYTISCHES MESSGERÄT MIT STECHHILFE**
ANALYTIC MEASURING DEVICE WITH A PUNCTURING AID
APPAREIL DE MESURE ANALYTIQUE AVEC ACCESSOIRE DE PIQUAGE

(30) Priorität: 28.11.1997 DE 19752688; 29.05.1998 DE 19824036
(43) Veröffentlichungstag der Anmeldung: 06.09.2000
(73) Patentinhaber: Roche Diagnostics GmbH, 68305 Mannheim (DE)
(72) Erfinder: KINTZIG, Hans, D-67311 Tiefenthal (DE); SCHABBACH, Michael, D-69493 Hirschberg (DE); MURAWSKI, Hans-Rüdiger, D-68623 Lampertheim (DE); OBERMEIER, Wolfgang, D-69120 Heidelberg (DE); MILTNER, Karl, D-67227 Frankenthal (DE)
(86) Internationale Anmeldenummer: PCT/EP1998/007706
(87) Internationale Veröffentlichungsnummer: WO 1999/027854

(56) Entgegenhaltungen:
- WO-A-88/00812
- DE-A- 4 234 553
- US-A- 4 637 403

## Beschreibung

Die Erfindung betrifft ein System zur Bestimmung der Anwesenheit oder des Gehalts eines Analyten in Blut, wobei das System ein Meßgerät zum Messen und Anzeigen der Änderung einer mit dem Analyten korrelierten, charakteristischen Eigenschaft eines Testelements sowie eine Stechhilfe für die Gewinnung des Bluts aus einer Körperpartie eines Probanden beinhaltet.

Die Bestimmung des Gehalts an bestimmten Analyten in Blut, beispielsweise von Glucose oder Lactat, erfordert die Gewinnung einer ausreichenden Probenmege (Blut) sowie die Bereitstellung eines geeigneten Meßsystems für den Analyten. Neben Arztpraxen und Labors führen vermehrt medizinische Laien solche Bestimmungen für den Eigengebrauch durch. Insbesondere für die Bestimmung und Kontrolle des Blutzuckerwertes, d. h. des Blutglucosegehaltes, bei Diabetikern, aber auch für die Ermittlung anderer Parameter wie Lactatgehalt oder Cholesterinspiegel, sind vom Probanden selbst anzuwendende Meßsysteme weit verbreitet.

Herkömmliche Meßsysteme enthalten oftmals Testelemente in Form sogenannter Teststreifen, die zusammen mit entsprechenden Meßgeräten die Bestimmung eines oder mehrerer Analyten in Blut erlauben. Daneben benötigt der Anwender weiterhin im Allgemeinen eine Lanzette, mit deren Hilfe die Haut an bestimmten Körperpartien, beispielsweise an der Fingerbeere oder dem Ohrläppchen, durchstochen und so Blut gewonnen werden kann, das für die Messung verwendet werden soll. Für eine komfortable Blutgewinnung bieten verschiedene Hersteller Stechhilfen an, welche die Lanzetten kontrolliert und geführt in die Haut stechen, um somit die Einstichtiefe zu kontrollieren und den Schmerz zu minimieren.

Da der Anwender zur Messung eines Analyten in Blut mehrere separate Komponenten benötigt (Testelemente, Meßgerät, Stechhilfe, Lanzetten etc.) und für eine Analyse "außer Haus", beispielsweise auf Reisen oder beim Sport, mit sich führen muß, ist es verständlich, daß vor allem Diabetiker, die zusätzlich auch noch Insulin und eine Spritze mit sich führen müssen, eine Reduzierung der mitzuführenden Einzelteile für wünschenswert erachten.

Deshalb mangelt es nicht an unterschiedlichen Versuchen, die Anzahl der Einzelteile zu reduzieren. Eine Lösung besteht darin, die benötigten Komponenten wie Meßgerät, Stechhilfe, Lanzetten und Teststreifen in ein gemeinsames Transportetui zu verpacken. Diese sind jedoch oft voluminös und beispielsweise für eine Aufbewahrung in einer Jacken- oder Hemdtasche zu groß und zu schwer.

Eine weitere Lösung besteht darin, möglichst viele der oben genannten Einzelkomponenten in ein Gerät zu integrieren. Die Integration eines Teststreifenbevorratungssystems in ein Meßgerät, wie sie in US 5,489,414 oder US 5,645,798 beschrieben ist, ist ein Beispiel hierfür.

Systeme, in denen Stechhilfe und Meßgerät zu einem Gerät verschmolzen sind, sind beispielsweise in US 5,029,583 und US 4,637,403 beschrieben. Durch diese integrierten Systeme ist zwar das Problem der Reduzierung der mit sich zu führenden Einzelteile im wesentlichen gelöst; die Handhabung solcher integrierten Systeme zur Blutgewinnung ist jedoch unbefriedigend, da die Systeme zumeist unhandlich und vergleichsweise schwer sind und dadurch das präzise Anvisieren eines Stechortes erschweren.

DE 42 34 553 offenbart ein System, bei welchem die Seiten des Messgeräts und der Stechhilfe nicht gleichermaßen zugänglich sind für die gewünschte Körperpartie, aus der Blut gewonnen werden soll.

Die Aufgabe der vorliegenden Erfindung war es, die Nachteile des Standes der Technik zu beseitigen. Insbesondere sollte ein System, welches Meßgerät und Stechhilfe in sich vereinigt, gefunden werden, das bei insgesamt reduzierter Zahl der mitzuführenden Einzelteile ein präzises Anvisieren des Stechortes erlaubt.

Die Aufgabe wird durch den Gegenstand der Erfindung, wie er in den Patentansprüchen charakterisiert ist, gelöst.

Das erfindungsgemäße System ist zur Bestimmung der Anwesenheit oder des Gehalts eines Analyten in Blut geeignet. Die Bestimmung der Anwesenheit eines Analyten kann beispielsweise für die qualitative Diagnose einer Infektion (z. B. mit Viren wie HIV oder HCV) oder die qualitative Erfassung eines bestimmten Körperzustandes (z. B. Schwangerschaft, Herzinfarkt) dienen. Die Bestimmung des Gehalts eines Analyten kann der Kontrolle eines Krankheitsverlaufs oder eines Therapieerfolges dienen und gibt detaillierte Auskunft über den Körperzustand eines Probanden. Beispiele hierfür sind die Messung des Blutglucosegehalts, der Lactat- oder Cholesterinkonzentration in Blut und dergleichen mehr. Vorzugsweise ist mit dem erfindungsgemäßen System die Bestimmung eines Parameters möglich. Die gleichzeitige Bestimmung mehrerer Parameter ist in einer weiteren Ausführungsform des erfindungsgemäßen Systems ebenfalls möglich.

Erfindungsgemäß erfolgt mit dem Meßgerät des Systems das Messen und Anzeigen der Änderung einer mit dem Analyten korrelierten, charakteristischen Eigenschaft eines Testelements. Das Testelement kann dabei in beliebiger, dem Fachmann an sich bekannter Form vorliegen, beispielsweise in Form eines Teststreifens oder einer Küvette, wobei vorzugsweise in oder auf dem Testelement die für die Nachweisreaktion erforderlichen Reagenzien enthalten sind. Mit Hilfe des Testelements ist es möglich, bei Anwesenheit eines oder mehrerer Analyten ein detektierbares Signal als charakteristische, mit dem Analyten korrelierte Eigenschaft des Testelements zu erzeugen. Beispielsweise seien genannt Farbänderungen in einer Nachweisreagenzienschicht, die photometrisch gemessen werden können, oder elektrische Ströme oder Potentialänderungen, zu deren Detektion Elektrodensysteme eingesetzt werden. Die so erzeugten Signale werden dann vom Meßgerät gemessen und ausgewertet, indem sie beispielsweise mit Kalibrationswerten verglichen werden. Je nach zu untersuchendem Analyten sind unterschiedliche Nachweis- und Bestimmungsprinzipien möglich, die sich einerseits durch das physikalischchemische Nachweisprinzip (z. B. Photometrie, Elektrochemie) und andererseits durch die (bio)-chemische Wechselwirkungen, die zu den charakteristischen, detektierbaren Veränderungen führen (z. B. Nachweis über enzymatische oder immunologische Reaktionen, Nukleinsäuresequenznachweis), unterscheiden.

Das erfindungsgemäße System enthält neben dem Meßgerät eine Stechhilfe, mit deren Hilfe eine Blutprobe eines Probanden gewonnen werden kann. Stechhilfen sind dem Fachmann beispielsweise aus EP-B 0 565 970 bekannt und werden von verschiedenen Anbietern in unterschiedlichen Formen kommerziell vertrieben. Sie dienen in Kombination mit Lanzetten der komfortablen, reproduzierbaren und möglichst schmerzarmen Gewinnung von Blut, beispielsweise aus der Fingerbeere oder dem Ohrläppchen.

Erfindungsgemäß ist in dem beanspruchten System die Stechhilfe direkt und lösbar mit dem Meßgerät verbunden. Unter direkter Verbindung soll dabei verstanden werden, daß neben der Stechhilfe und dem Meßgerät keine zusätzlichen, separaten Vorrichtungen, wie z. B. Taschen, Hüllen, Bänder oder Etuis, zum Verbinden von Stechhilfe und Meßgerät erforderlich sind
Vielmehr enthält entweder das Meßgerät oder die Stechhilfe oder beide Komponenten entsprechende, vorzugsweise aufeinander abgestimmte und aneinander angepaßte Verbindungsmittel, die ein lösbares Verbinden der beiden Komponenten des Systems erlauben. Mögliche Verbindungsmittel sind Steckverbindungen, Klemmverbindungen,Verbindungen über profilierte Schienen, Verbindung mittels Magneten oder Klettverschlüssen. Vorzugsweise wird die Verbindung über Steckverbindungen bewerkstelligt, wobei die Verbindung mit Hilfe eines Klipps besonders bevorzugt ist.

Die erfindungsgemäße Verbindung von Stechhilfe und Meßgerät ist leicht und schnell lösbar und wieder schließbar, und erlaubt somit eine bequeme Handhabung des erfindungsgemäßen Systems, was dessen Akzeptanz bei den Benutzern erhöht.

Da für eine bevorzugte Ausführungsform der Erfindung die Gewinnung des Blutes eines Probanden durch die Stechhilfe sowohl im losgelösten als auch im mit dem Meßgerät verbundenen Zustand gleichermaßen möglich sein sollte, hat sich eine stabile, das heißt den beim Benutzen der Stechhilfe entstehenden Scherkräften auf die Verbindung widerstehende Verbindung als besonders vorteilhaft erwiesen. Besonders bevorzugt ist die Verbindung mit Hilfe eines Klipps. Klipps sind in unterschiedlichen Formen, Materialien und Ausführungen beispielsweise für Schreibgeräte wie Füllfederhalter, Kugelschreiber oder Faserschreiber bekannt.

Ganz besonders bevorzugt ist der Klipp an der Stechhilfe, welche ganz besonders bevorzugt eine im wesentlichen zylindrische, kugelschreiberähnliche Form wie in EP-B 0 565 970 besitzt, angebracht und das Meßgerät, welches vorzugsweise im wesentlichen die Form eines flachen Quaders hat, enthält eine entsprechend geformte Ausnehmung, besonders bevorzugt an einer seiner schmalen Seitenflächen, welche die paßgenaue Aufnahme des Klipps und somit die stabile, lösbare und direkte Verbindung von Stechhilfe und Meßgerät erlaubt. Vorzugsweise sind der Klipp und die entsprechende Ausnehmung zur Aufnahme des Klipps so geformt, daß das Erreichen der stabilen Verbindungsposition taktil oder akustisch von Anwender wahrzunehmen ist. Durch die Kombination von Klipp an der Stechhilfe und im wesentlichen dazu komplementäre Ausnehmung im Meßgerätegehäuse wird eine eindeutige, orientierte und geführte Verbindung der beiden erfindungsgemäßen Systemkomponenten erzielt, wobei das Spiel zwischen Stechhilfe und Meßgerät optimiert ist, um einerseits eine stabile, den Scherkräften widerstehende Verbindung zu schaffen, die andererseits leicht und schnell zu lösen ist. Besonders bevorzugt kommt die Stechhilfe durch die Verbindung mit dem Meßgerät im wesentlichen an dessen schmaler Seite zu liegen.

Stechhilfe und Meßgerätegehäuse sind vorzugsweise so gestaltet, daß sich eine kompakte Gesamtform ergibt. Beispielsweise kann im Meßgerätegehäuse eine Aussparung in Form einer Mulde vorgesehen sein, welche die Stechhilfe zumindest teilweise umschließt. Neben der Kompaktheit der Anordnung wird dadurch auch eine zusätzliche Stabilität der lösbaren Verbindung von Stechhilfe und Meßgerät erreicht.

Der Klipp zur Verbindung von Stechhilfe und Meßgerät kann sowohl aus dem Gehäusematerial der Stechhilfe bzw. des Meßgeräts als auch aus einem anderen geeigneten Material geschaffen sein. Geeignete Materialien sind beispielsweise Metalle, Legierungen oder Kunststoffe oder Kombinationen hiervon, z. B. kunststoffbeschichtete Metalle oder metallisierte Kunststoffe. Der Klipp kann dabei sowohl Bestandteil des Gehäuses sein, welches beispielsweise als ein Spritzgußteil gefertigt ist, oder ein separates, jedoch fest mit dem Gehäuse verbundenes Teil.

Ebenfalls als bevorzugt hat sich die Verbindung von Stechhilfe und Meßgerät durch Ausformungen des Meßgerätegehäuses herausgestellt. Beispielsweise kann das Meßgerät an einer, vorzugsweise einer schmalen Gehäuseseite flexible Klemmbacken enthalten, mit denen die Stechhilfe teilweise umschlossen und so an das Meßgerät lösbar fixiert werden kann. In diesem Fall kann darauf verzichtet werden, an der Stechhilfe eigene Vorrichtungen zum Befestigen am Meßgerät anzubringen. Die Mittel zur lösbaren Verbindung von Stechhilfe und Meßgerät können in dieser bevorzugten Ausführungsform integraler Bestandteil des Meßgerätegehäuses sein, beispielsweise bei der Fertigung in Spritzgußtechnik in dieses eingebracht sein. Es ist jedoch auch möglich, daß die besagten Mittel als separates, jedoch fest mit dem Gehäuse verbundenes Teil , ausgeformt sind.

Durch das erfindungsgemäße System wird dem Benutzer ein kompaktes Stechhilfe-Meßgeräte-System zur Verfügung gestellt, welches die Zahl der Einzelteile, die für die Gewinnung von Blut und die anschließende Messung eines Blutparameters vom Benutzter mitzuführen sind, reduziert. Vorteilhaft ist weiterhin, daß es dem Anwender überlassen bleibt, ob er die Stechhilfe im mit dem Meßgerät verbundenen oder im vom Meßgerät gelösten Zustand benutzen will, wodurch eine hohe Flexibilität bei der Anwendung erreicht wird. Zudem ist eine sichere, hygienische Reinigung der Stechhilfe möglich, ohne daß die Gefahr einer Schädigung des Meßgerätes besteht.

Die Erfindung wird durch die nachfolgenden Figuren 1 bis 5 näher erläutert.
In Figur 1 ist schematisch eine Aufsicht von oben auf eine bevorzugte Ausführungsform des erfindungsgemäßen Systems zu sehen.
Figur 2 stellt eine schematische Aufsicht aus Richtung A auf das in Figur 1 gezeigte erfindungsgemäße System dar.
In Figur 3 ist der Verbindungsmechanismus für Stechhilfe und Meßgerät des erfindungsgemäßen Systems gemäß Figur 1 schematisch anhand einer teilweisen Schnittansicht dargestellt.
In Figur 4 ist anhand von 3 Teilfiguren (A bis C) eine zu den Figuren 1 bis 3 alternative, bevorzugte Ausführungsform des erfindungsgemäßen Systems abgebildet.
Figur 5 zeigt ebenfalls anhand von 3 Teilfiguren (A bis C) eine zu den Figuren 1 bis 4 alternative, bevorzugte Ausführungsform des erfindungsgemäßen Systems.

Die Ziffern in den Figuren bedeuten:
- 1: Meßgerät
- 2: Stechhilfe
- 3: Anzeige (Display)
- 4: Bedienelement für das Meßgerät
- 5: Bedienelement für die Stechhilfe
- 6: Verbindungselement 1 (Klipp)
- 7: Verbindungselement 2 (komplementäre Ausnehmung)
- 8: Gehäusewand des Meßgeräts 1
- 9: Klemmbacken

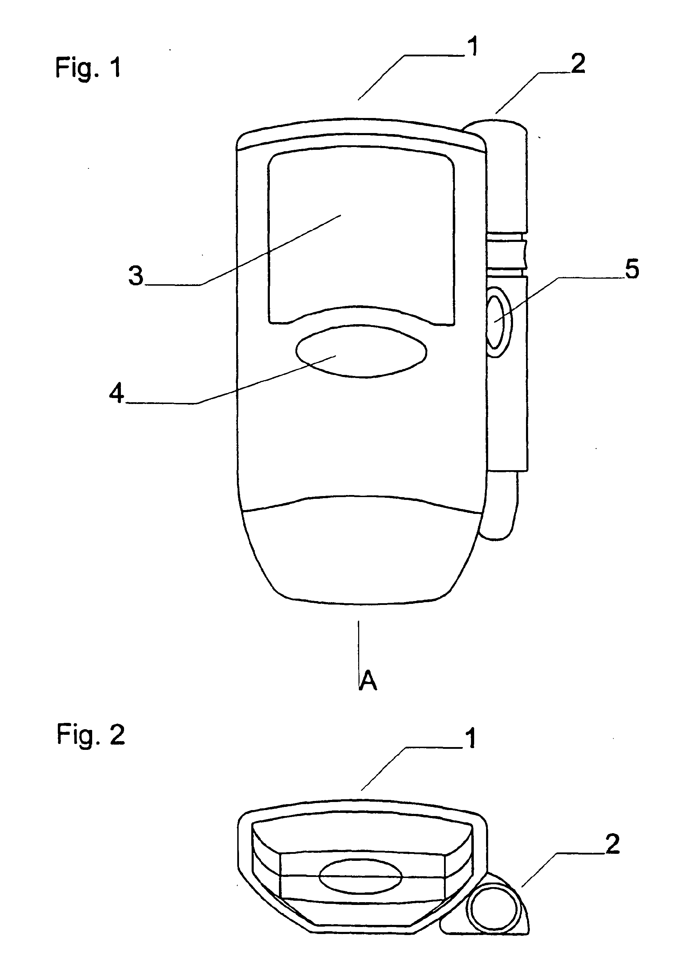

Das in den Figuren 1 bis 3 dargestellte, eine besonders bevorzugte Ausführungsform der Erfindung repräsentierende System besteht aus einem Meßgerät 1 und einer Stechhilfe 2, die direkt und lösbar miteinander verbunden sind. Dabei kommt die Stechhilfe 2 im wesentlichen seitlich neben dem Meßgerät 1 zu liegen.

Das Meßgerät 1 ist vorzugsweise so dimensioniert, daß es bequem in einer Hand gehalten werden kann. Die Stechhilfe 2 hat im wesentlichen die Form eines Füllfederhalters und ist in seiner Größe (Länge, Durchmesser) im wesentlichen dem Meßgerät 1 angepaßt, so daß für den Transport und die Benutzung des Systems keine überstehenden oder hervorragende Teile vorhanden sind.

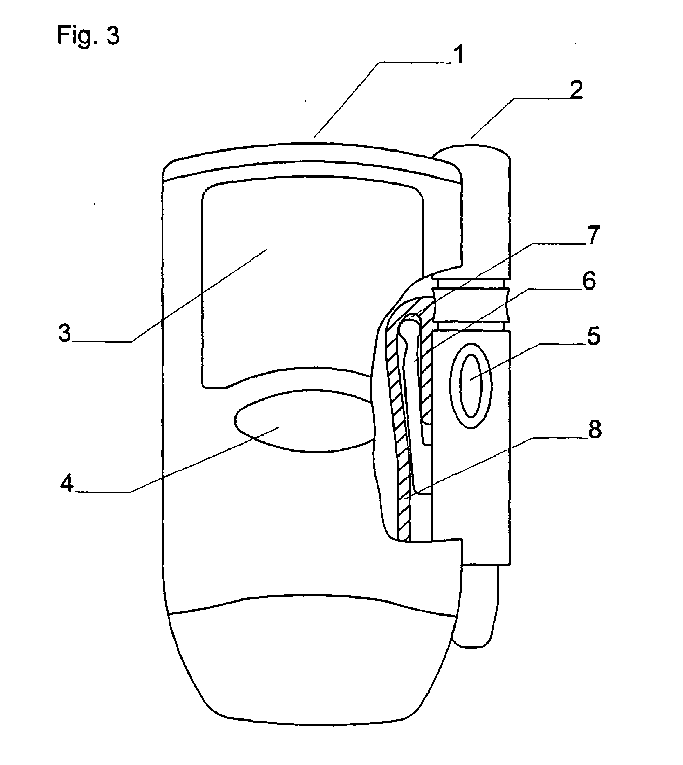

Das Meßgerät 1 enthält alle funktionsnotwendigen Bestandteile, von denen in den Figuren 1 und 3 lediglich die Anzeige (Display) 3 sowie ein Bedienelement 4 schematisch dargestellt sind. Die Testelemente, die mit dem Meßgerät 1 vermessen werden sollen, werden von der Seite A in Figur 1 in das Gerät 1 eingeführt oder können alternativ vom Gerät 1, beispielsweise aus einem integrierten Testelementemagazin, zur Verfügung gestellt werden.

Die Stechhilfe 2 enthält ebenfalls alle funktionsnotwendigen Bestandteile, von denen lediglich ein Bedienelement 5 schematisch dargestellt ist. Die Lanzetten zur Blutgewinnung werden von Seite A (Fig. 1) in die Stechhilfe 2 eingeführt.

In Figur 3 ist schematisch eine Detailansicht der Verbindungselemente 1 (Klipp 6) und 2 (komplementäre Aussparung 7) abgebildet, wozu ein Teil der Meßgeräteoberseite ausgelassen ist. Der Klipp 6 paßt genau in die komplementäre Aussparung 7, die von der Gehäusewand 8 des Meßgeräts 1 gebildet wird.

Bei der Benutzung der Stechhilfe 2 wird sie der Benutzer in der Regel vom Meßgerät 1 lösen, um eine möglichst präzise und schmerzarme Blutgewinnung durchzuführen. Dazu muß eine entsprechende Lanzette in die Stechhilfe 2 eingeführt werden, die Stechhilfe 2 beispielsweise durch Spannen einer Feder funktionsbereit gemacht werden und anschließend an die gewünschte Körperpartie, beispielsweise die Fingerbeere oder das Ohrläppchen, gebracht werden, aus der Blut gewonnen werden soll. Durch Betätigen des Bedienelements 5 wird die Lanzette in die gewünschte Körperpartie gestochen und somit Blut gewonnen.

Das Blut wird anschließend auf den Probenaufgabebezirk eines Testelements aufgebracht, welches sich vorzugsweise bereits im Meßgerät 1 befindet, wobei zumindest der Probenaufgabebezirk des Testelements aus dem Meßgerät 1 herausragt. Durch Betätigen des Bedienelements 4 wird die eigentliche Messung gestartet. In der Anzeige 3 erscheint nach Abschluß der Nachweisreaktion auf dem Testelement der Meßwert der Analytbestimmung.

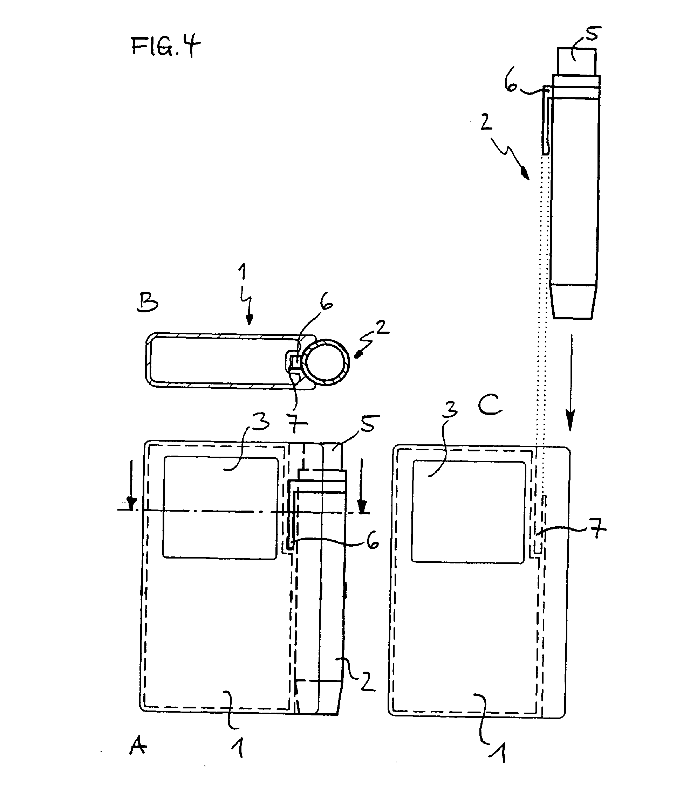

Bei der in Figur 4 dargestellten, weiteren bevorzugten Ausführungsform des erfindungsgemäßen Systems aus Stechhilfe 2 und Meßgerät 1 wird die lösbare Verbindung dieser beiden Systemkomponenten ebenfalls durch einen Klipp 6 an der Stechhilfe 2 und eine entsprechende, komplementäre Ausnehmung 7 am Meßgerät 1 realisiert. Die Ausnehmung 7 befindet sich dabei in einer Seitenwand des Meßgerätegehäuses, welche zur Stabilisierung der Verbindung von Meßgerät 1 und Stechhilfe 2 den Konturen der Stechhilfe angepaßt ist.

In Figur 4 A ist anhand einer Aufsicht gezeigt, wie sich Meßgerät 1 und Stechhilfe 2 im miteinander verbundenen Zustand befinden. In Figur 4 B ist ein Schnitt durch die in Figur 4 A angegebene, mit Pfeilen markierte Ebene von Meßgerät 1 und Stechhilfe 2 abgebildet. In dieser Schnittdarstellung ist gut zu erkennen, wie die Seitenwand des Meßgerätegehäuses an die Kontur der Stechhilfe 2 angepaßt ist.

Figur 4 C verdeutlicht, wie die Stechhilfe 2 an das Meßgerät 1 angedockt wird. Die Stechhilfe 2 wird parallel zum Meßgerät 1 mit dem Klipp 6 zur Gehäuseseite orientiert, welche die zum Klipp 6 komplementäre Ausnehmung 7 enthält, und durch Einführen des Klipps 6 in die Ausnehmung 7 mit dem Meßgerät 1 verbunden. Klipp 6, Ausnehmung 7 und die Anpassung der Seitenwand des Meßgeräts 1 an die Stechhilfe 2 sorgen dafür, daß die Verbindung stabil, aber wieder lösbar ist. Das Lösen der Verbindung erfolgt dabei in umgekehrter Reihenfolge wie das Herstellen der Verbindung.

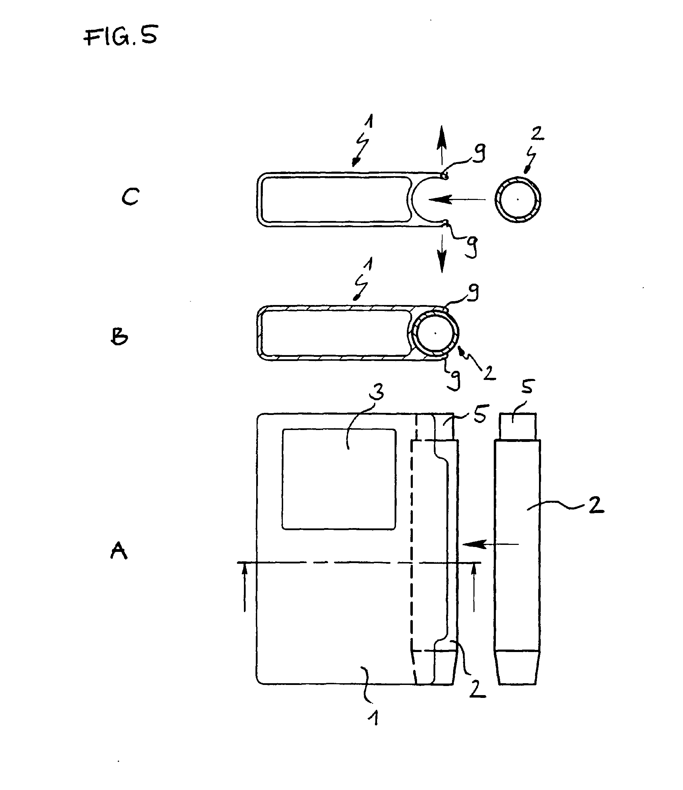

In Figur 5 ist schließlich ein alternativer Verbindungsmechanismus für Stechhilfe 2 und Meßgerät 1 gezeigt. Die Stechhilfe 2 enthält dabei keine speziellen Vorrichtungen, die eine Verbindung mit dem Meßgerät erlauben. Vielmehr wird die Verbindung durch eine spezielle Ausformung einer der seitlichen Gehäusewände des Meßgeräts 1 bewirkt. Diese Gehäusewand enthält Klemmbacken 9, die in der gezeigten bevorzugten Ausführungsform die Stechhilfe 2, welche einen runden Querschnitt aufweist (Figur 5 B und C), teilweise umschließen und so leicht lösbar festhalten. Das Material, aus dem die Backen 9 sind, ist dabei zum einen so flexibel, daß es zum Aufnehmen der Stechhilfe 2 auseinandergedrückt werden kann, und zum anderen so steif, daß es die Stechhilfe 2, wenn sie erst einmal von den Backen 9 teilweise umschlossen ist und am Meßgerät 1 anliegt, fixiert.

Figur 5 A deutet in einer Aufsicht an, wie die Stechhilfe 2 seitlich an das Meßgerät 1 angenähert wird und durch die Klemmbacken 9 am Meßgerät 1 gehalten wird. In Figur 5 B ist ein schematischer Schnitt durch die in Figur 5 A angegebene, mit Pfeilen markierte Ebene von Meßgerät 1 und Stechhilfe 2 abgebildet. In dieser Schnittdarstellung ist gut zu erkennen, wie die Klemmbacken 9, die Bestandteile der Gehäusewand des Meßgeräts 1 sind, die Stechhilfe 2 umschließen. Figur 5 C ist wie Figur 5 B eine schematische Schnittansicht von Stechhilfe 2 und Meßgerät, wobei hier anhand der eingezeichneten Pfeile verdeutlicht werden soll, wie sich die Backen 9 des Meßgerätegehäuses beim Einführen der Stechhilfe 2 auseinanderbewegen.

## Patentansprüche

1. System zur Bestimmung der Anwesenheit oder des Gehalts eines Analyten in Blut, beinhaltend ein Messgerät (1) zum Messen und Anzeigen der Änderungen einer mit dem Analyten korrelierten, charakterisierten Eigenschaft eines Testelements, wobei das Messgerät (1) eine Seite (A) des Messgerätes (1) aufweist und ein Testelement zur Analyse einer Blutprobe von der Seite A in das Messgerät (1) einführbar oder vom Messgerät (1) von der seite A zur Verfügung stellbar ist, und eine Stechhilfe (2) für die Gewinnung des Bluts aus einer Körperpartie eines Probanden, wobei die Stechhilfe (2) eine Seite (A) der Stechhilfe (2) aufweist wobei eine Lanzette von der seite A in die Stechhilfe einführbar ist und
das Messgerät und die Stechhilfe direkt und lösbar miteinander verbunden sind, wobei die Stechhilfe (2) im Wesentlichen seitlich neben dem Messgerät (1) angeordnet ist, so dass, wenn die Stechhilfe und das Messgerät in verbundenem Zustand benutzt werden, die Seite (A) des Messgeräts und die Seite (A) der Stechhilfe (2) der gewünschten Körperpartie, aus der Blut gewonnen werden soll, zugewandt sind und für die besagte Körperpartie zugänglich sind.

2. System gemäß Anspruch 1, **dadurch gekennzeichnet, dass** die Stechhilfe mit dem Messgerät direkt und lösbar über einen Klipp verbunden ist.

3. System gemäß einem der Ansprüche 1 bis 2, **dadurch gekennzeichnet, dass** sowohl am Messgerät als auch an der Stechhilfe Vorrichtungen vorhanden sind, die eine lösbare, direkte Verbindung von Messgerät und Stechhilfe ermöglichen.

4. System gemäß einem der Ansprüche 1 bis 3, **dadurch gekennzeichnet, dass** die Gewinnung des Bluts aus einer Körperpartie des Probanten mit Hilfe der Stechhilfe sowohl im mit dem Messgerät verbundenen, als auch im vom Messgerät gelösten Zustand der Stechhilfe möglich ist.

## Claims

1. System for determining the presence or the content of an analyte in blood comprising a measuring instrument (1) for measuring and displaying the change of a characteristic property of a test element which correlates with the analyte wherein the measuring instrument (1) has a side (A) of the measuring instrument (1) and a test element for analysing a blood sample can be inserted from side (A) into the measuring instrument (1) or can be provided by the measuring instrument (1) from side (A) and a lancing device (2) for collecting blood from a body region of a test person, wherein the lancing device (2) has a side (A) of the lancing device (2), wherein a lancet can be inserted into the lancing device from side (A) and the measuring instrument (1) and the lancing device (2) are directly and detachably connected to one another, the lancing device (2) being located essentially laterally next to the measuring instrument so that when the lancing device and the measuring instrument are used in a connected state, side (A) of the measuring instrument and side (A) of the lancing device (2) face the desired body region from which blood should be collected and are accessible to the said body region.

2. System according to claim 1, **characterized in that** the lancing device is connected directly and detachably to the measuring instrument by means of a clip.

3. System according to one of the claims 1 to 2, **characterized in that** devices are present on the measuring instrument as well as on the lancing device which enable the measuring instrument and lancing device to be connected detachably and directly.

4. System according to one of the claims 1 to 3, **characterized in that** the blood can be collected from a body region of the test person with the aid of a lancing device where the lancing device can either be connected to the measuring device or detached from the measuring instrument.

## Revendications

1. Système de détermination de la présence dans le sang, ou du taux sanguin d'un analyte, comprenant un instrument de mesure (1) destiné à mesurer une propriété **caractérisée** qui est en corrélation avec l'analyte, et à indiquer les modifications de cette propriété, dans lequel l'instrument de mesure (1) présente une face (A), et un élément de test destiné à analyser un échantillon de sang peut être introduit par la face (A) de l'instrument de mesure (1) ou peut être disponible à la face (A) de l'instrument de mesure (1), et un auxiliaire de piqûre (2) destiné au prélèvement de sang à partir d'une partie corporelle d'un sujet, l'auxiliaire de piqûre (2) présentant une face (A), une lancette étant apte à être introduite par la face (A) de l'auxiliaire de piqûre (2), et
l'instrument de mesure et l'auxiliaire de piqûre sont reliés entre eux directement et de façon détachable, l'auxiliaire de piqûre (2) étant disposé essentiellement sur le côté de l'instrument de mesure (1), de sorte que, lorsque l'instrument de mesure et l'auxiliaire de piqûre sont utilisés en étant reliés entre eux, la face (A) de l'instrument de mesure et la face (A) de l'auxiliaire de piqûre (2) sont tournés vers la partie corporelle dans laquelle le sang doit être prélevé, et sont accessibles pour ladite partie corporelle.

2. Système selon la revendication 1, **caractérisé en ce que** l'auxiliaire de piqûre est relié à l'instrument de mesure directement et de façon détachable au moyen d'un clip.

3. Système selon l'une quelconque des revendications 1 à 2, **caractérisé en ce que** sur l'instrument de mesure aussi bien que sur l'auxiliaire de piqûre, sont présents des dispositifs qui permettent de réaliser une liaison directe et détachable entre l'instrument de mesure et l'auxiliaire de piqûre.

4. Système selon l'une quelconque des revendications 1 à 3, **caractérisé en ce que** le prélèvement du sang à partir d'une partie corporelle d'un sujet est réalisable à l'aide de l'auxiliaire de piqûre lorsque l'auxiliaire de piqûre est relié à l'instrument de mesure aussi bien que lorsqu'il est détaché de l'instrument de mesure.
